Europäisches Patentamt

European Patent Office

Office européen des brevets

(19)

(11) Veröffentlichungsnummer: **0 161 386**
A2

# EUROPÄISCHE PATENTANMELDUNG

(12)

(21) Anmeldenummer: 85100483.8

(22) Anmeldetag: 18.01.85

(51) Int. Cl.⁴: **A 61 K 6/08,** C 09 J 3/14, C 08 F 220/00

(30) Priorität: 14.04.84 DE 3414164

(43) Veröffentlichungstag der Anmeldung: 21.11.85 Patentblatt 85/47

(84) Benannte Vertragsstaaten: **AT CH DE FR GB LI SE**

(71) Anmelder: **Kulzer & Co. GmbH, Philipp-Reis-Strasse 8, D-6393 Wehrheim (TS.)1 (DE)**

(72) Erfinder: **Janda, Ralf, Dr., Seulbergerstrasse 19, D-6380 Bad Homburg (DE)**
Erfinder: **Eppinger, Bernhard, Ringstrasse 26, D-6393 Wehrheim 3 (DE)**

(74) Vertreter: **Heinen, Gerhard, Dr., Heraeusstrasse 12-14, D-6450 Hanau/Main (DE)**

(54) Photopolymerisierbarer dentaler Haftvermittler-Lack.

(57) Mit einem Haftvermittler-Lack aus einer Lösung von Tetramethacryloyloxyäthylpyrophosphat und eines Photopolymerisationskatalysators in Aceton kann unter Verwendung eines photoplymerisierbaren Versiegelungsmaterials die Haftung eines Zahnfüllungsmaterials auf Kunststoff-Basis am Dentin verbessert werden.

EP 0 161 386 A2

0161386

Hanau, 11. April 1984
ZPL-Pr/ha

Kulzer & Co. GmbH

Patentanmeldung

"Photopolymerisierbarer dentaler Haftvermittler-Lack"

Die Erfindung betrifft einen dentalen Haftvermittler-Lack
aus einer Lösung eines Methacryloyloxyäthylesters einer Phosphorsäure in einem flüchtigen organischen Lösungsmittel.

Beim Füllen von Zahnkavitäten mit einem polymerisierbaren
Zahnfüllungsmaterial auf Kunststoff-Basis wird zur Verbesserung
der Haftung zwischen Zahnschmelz und Kunststoff der Zahnschmelz
vor dem Legen der Füllung mit einer sauren Ätzlösung, zum
Beispiel einer verdünnten Phosphorsäure-Lösung, behandelt
und gegebenenfalls dann noch mit einem dünnflüssigen, meist
monomere Dimethacrylate enthaltenden chemisch oder durch
Photopolymerisation auszuhärtenden Versiegelungsmaterial
bestrichen. Wegen ihrer guten physikalischen Eigenschaften
haben sich Versiegelungsmaterialien, die zusätzlich zu den
Monomeren einen feinteiligen anorganischen Füllstoff enthalten,
besonders bewährt. Ihre Haftung beruht auf einer mechanischen
Verankerung im geätzten Zahnschmelz.

Ein Ätzen des Dentins empfiehlt sich aus zahnärztlicher Sicht
jedoch nicht. Daher werden, wenn eine Verankerung durch Schmelzätzung nicht möglich ist, zum Beispiel bei Zahnhalskavitäten,
zur Verbesserung der Haftung zwischen Dentin und Kunststoff
Haftmittel verwendet. Eine Vielzahl solcher dentaler Haftmittel
ist bekannt.

In Adhesive Restorative Dental Materials 1961, 195-198, wird
über die haftverbessernde Wirkung von Bis-(methacryloyloxy-
äthyl)-hydrogenphosphat und einigen Methacryloyloxy-Gruppen
enthaltenden Dihydrogenphosphaten berichtet.

- 2 -

Aus der europäischen Patentanmeldung 88 527 ist ein die Haftung verbesserndes Material ("adhesion promotor"), das aus einer Lösung eines mindestens drei Methacryloyloxy-Gruppen aufweisenden Dihydrogenphosphats in einem flüchtigen organischen Lösungsmittel, besonders einem Alkanol, besteht, bekannt. Zusammen mit diesem Material kann, um die Haftung des Zahnfüllungsmaterials an der Zahnsubstanz zu verstärken, ein Haft- oder Versiegelungsmaterial ("intermediate bonding resin") verwendet werden.

Aus den veröffentlichten japanischen Patentanmeldungen 57/56 490 und 57/167 364 ist die Verwendung von Tetramethacryloyloxyäthylpyrophosphat in Dentalmaterialien bekannt (Chemical Abstracts Vol. 98, 1983, 34 758 und 204 455).

Es ist die Aufgabe der Erfindung, ein Mittel zu finden, das durch chemische Bindung die Haftung eines photopolymerisierbaren Versiegelungsmaterials am Dentin bewirkt.

Diese Aufgabe wird erfindungsgemäß durch einen Haftvermittler-Lack, der aus einer Lösung von Tetramethacryloyloxyäthylpyrophosphat und eines Photopolymerisationskatalysators in Aceton besteht, gelöst.

Vorzugsweise enthält der Haftvermittler-Lack 10 - 30 Gewichts-% des Pyrophosphats, besonders etwa 15 - 20 Gewichts-%.

Als Photopolymerisationskatalysatoren haben sich die aus der GB-PS 1 408 265 bekannten Gemische aus Ketonen und Aminen, besonders aus Campherchinon und Aminen oder aus Campherchinon, Benzoylverbindungen, zum Beispiel Benzilacetale oder Benzoylalkanole, und Aminen, zum Beispiel 4-Dimethylaminobenzoesäure-2-butoxyäthylester, bewährt.

Vorzugsweise enthält der Haftvermittler-Lack jeweils 0,1 - 0,5 Gewichts-% des Campherchinons, des Amins und der Benzoylverbindung.

Durch die Anwendung des Haftvermittler-Lackes gemäß der Erfindung
zusammen mit einem photopolymerisierbaren Versiegelungsmaterial
läßt sich eine haft- und dauerfeste Verbindung zwischen Dentin
und dem Zahnfüllungsmaterial auf Kunststoff-Basis schaffen.

Das in dem in dünner Schicht auf das Dentin gestrichenen
Haftvermittler-Lack vorhandene Aceton bewirkt eine gute Benetzung
der Dentinoberfläche und eine gleichmäßige Verteilung des
Lackes. Ein weiterer Vorteil des Acetons ist es, daß mit
seinem Verdunsten auch am Dentin noch vorhandene Spuren von
Feuchtigkeit entfernt werden. Das nach dem Verdunsten des
Acetons aufzutragende einen Photopolymerisationskatalysator
enthaltende Versiegelungsmaterial wird durch Bestrahlung
mit UV-Licht, vorzugsweise jedoch durch Bestrahlung mit sichtbarem Licht ausgehärtet.

Mit dem einen Photopolymerisationskatalysator enthaltenden
Haftvermittler-Lack gemäß der Erfindung wird - wahrscheinlich
aufgrund der vollständigen Copolymerisation des Pyrophosphats
mit den Monomeren des Versiegelungsmaterials - eine höhere
Haftfestigkeit als mit einem keinen Katalysator dieser Art
enthaltenden Lack erreicht.

Zur näheren Erläuterung wird in dem folgenden Beispiel die
Herstellung eines Haftvermittler-Lackes gemäß der Erfindung
beschrieben.

Die mit diesem Haftvermittler-Lack unter Verwendung eines
photopolymerisierbaren Versiegelungsmaterials erreichte Haftfestigkeit zwischen Dentin und Kunststoff-Zahnfüllung wird
bestimmt.

Beispiel

Durch Vermischen von

       79,5 g Aceton, absolut

       20,0 g Tetramethacryloyloxyäthylpyrophosphat

0,1 g   1,2-Diphenyl-2,2-dimethoxyäthanon

0,3 g   Campherchinon und

0,1 g   4-Dimethylaminobenzoesäure-2-butoxyäthylester

wird ein Haftvermittler-Lack hergestellt.

Dieser Lack wird auf die mit einer Diamantsäge plan gesägte, polierte und getrocknete Dentinoberfläche von extrahierten Zähnen aufgestrichen. Nach dem Verdunsten des Acetons wird auf die erhaltene Lack-Schicht eine dünne Schicht des photopolymerisierbaren Versiegelungsmaterials Durafill bond der Firma Kulzer aufgetragen, durch 20 Sekunden langes Bestrahlen mit dem Halogenlichtgerät der Firma Kulzer (Translux) ausgehärtet und mit dem photopolymerisierbaren Zahnfüllungsmaterial Durafill der Firma Kulzer bedeckt, das durch 20 Sekunden langes Bestrahlen mit dem Halogenlichtgerät ausgehärtet wird.

Die so behandelten Zähne werden zur Bestimmung der Haftfestigkeit zwischen Dentin und Kunststoff Abscherversuchen unterworfen. Die dabei unmittelbar nach Aushärtung des Zahnfüllungsmaterials, nach 7tägiger und nach 40tägiger Lagerung in 37 °C warmem Wasser erhaltenen Werte werden in der Tabelle angegeben.

Tabelle

|  | unmittelbar nach Aushärtung | nach Lagerung in Wasser, 37 °C | |
|---|---|---|---|
|  |  | 7 Tage | 40 Tage |
| Haftfestigkeit $(N/mm^2)$ | 7,4 | 5,4 | 4,3 |

Hanau, 11. April 1984
ZPL-Pr/ha


Kulzer & Co. GmbH

Patentanmeldung

"Photopolymerisierbarer dentaler Haftvermittler-Lack"


Patentansprüche

1. Dentaler Haftvermittler-Lack aus einer Lösung eines Methacryloyloxyäthylesters einer Phosphorsäure in einem flüchtigen organischen Lösungsmittel, dadurch gekennzeichnet, daß er aus einer Lösung von Tetramethacryloyloxyäthylpyrophosphat und eines Photopolymerisationskatalysators in Aceton besteht.

2. Haftvermittler-Lack nach Anspruch 1, dadurch gekennzeichnet, daß er 10 - 30 Gewichts-% des Pyrophosphats enthält.

3. Haftvermittler-Lack nach Anspruch 2, dadurch gekennzeichnet, daß er 15 - 20 Gewichts-% des Pyrophosphats enthält.

4. Haftvermittler-Lack nach einem der Ansprüche 1 - 3, dadurch gekennzeichnet, daß der Photopolymerisationskatalysator aus Keton und Amin besteht.

5. Haftvermittler-Lack nach Anspruch 4, dadurch gekennzeichnet, daß der Photopolymerisationskatalysator aus Campherchinon und Amin besteht.

6. Haftvermittler-Lack nach Anspruch 4, dadurch gekennzeichnet, daß der Photopolymerisationskatalysator aus Campherchinon, Benzoylverbindung und Amin besteht.